# EUROPEAN PATENT SPECIFICATION

(11) **EP 1 699 528 B1**
(45) Date of publication and mention of the grant of the patent: **27.08.2008**
(21) Application number: 04805965.3
(22) Date of filing: 10.12.2004
(51) Int. Cl.: A61Q 17/04, A61K 8/25, A61K 8/27, A61K 8/29, A61K 8/58, A61K 8/892

(54) **METAL OXIDE DISPERSIONS**
METALLOXIDDISPERSIONEN
DISPERSIONS D'OXYDE METALLIQUE

(30) Priority: 11.12.2003 GB 0328693
(43) Date of publication of application: 13.09.2006
(73) Proprietor: CRODA INTERNATIONAL PLC, Goole North Humberside DN14 9AA (GB); Croda Uniqema Inc, Chicago IL 60609 (US)
(72) Inventor: KESSELL, Lorna, Margaret, North Yorkshire DL6 3DP (GB); LYTH, Philip, Laurence, Cleveland TS17 5DN (GB); GORMLEY, John, L., Midland Park, NJ 07432 (US)
(74) Representative: Humphries, Martyn
(86) International application number: PCT/GB2004/005145
(87) International publication number: WO 2005/055968

(56) References cited:
- EP-A- 0 953 336
- WO-A-03/105790
- US-A- 6 124 490
- US-A1- 2002 054 999
- ANONYMOUS: "Arlatone V-175" UNIQEMA, [Online] XP002319957 Retrieved from the Internet: URL:http://web.archive.org/web/*/http://ww w.uniqema.com/pc/lit/americas/pc14/pc14.pd f> [retrieved on 2005-03-02]

## Description

### Field of Invention

This invention relates to dispersions of metal oxides and in particular to dispersions of metal oxides in a siloxane fluid dispersing medium having a polysiloxane containing a carboxyl group as dispersing agent.

### Background

Metal oxides such as titanium dioxide, zinc oxide and iron oxides have been employed as attenuators of ultraviolet light in applications such as sunscreens, plastics films and resins. Generally, metal oxides which are useful in these applications have an average primary particle size less of than 200 nm. Dispersions of such metal oxides in certain oily media and in water are known and these dispersions have been used to formulate products such as sunscreening creams and lotions. The availability of the metal oxide in the form of a dispersion which is subsequently mixed with other conventional formulation ingredients to prepare a product has been shown to be advantageous in preparing the products.

The use of siloxane or silicone based oils in cosmetics has become popular because they can produce an improved skin feel. Hence, metal oxide dispersions in siloxane based dispersing media are desirable. Such dispersions have been difficult to produce.

### Prior Art

EP-0953336-A discloses a method for producing a dispersion of ultraviolet shielding fine particles in a silicone oil using a modified silicone or reactive silicone dispersant and a mill or high pressure dispersion treatment. EP-0953336-A is particularly directed to the use of oxazoline-modified silicones, amino-modified silicones and polyether-modified silicones. EP-0953336-A discloses a maximum concentration of 40% by weight of particles, although the examples in the patent specification contain significantly lower amounts of particles. There is a need for a siloxane or silicone based dispersion containing a higher concentration of particles, in order to improve the flexibility to the sunscreen formulator. There is also a requirement for such a dispersion to have improved properties, such as stability and optical characteristics.

### Summary of the Invention

We have now surprisingly discovered a dispersion, which overcomes or significantly reduces at least one of the aforementioned problems.

Accordingly the present invention provides a dispersion comprising particles of metal oxide having an average particle size of less than 200 nm dispersed in a siloxane fluid and 1 to 60% by weigh based on the weight of metal oxide of a dispersing agent which is a polysiloxane wherein (i) the polysiloxane comprises in the range from 0.1 to 3 carboxyl groups, and (ii) the ratio of non-carboxyl group containing monomer units to carboxyl group containing monomer units in the polysiloxane is in the range from 40 to 150:1.

The invention further provides a method of preparing a dispersion of metal oxide which comprises milling with a particulate grinding medium particles of metal oxide in a siloxane fluid in the presence 1 to 60% by weight based on the weight of metal oxide of dispersing agent which is a polysiloxane wherein (i) the polysiloxane comprises in the range from 0.1 to 3 carboxyl groups, and (ii) the ratio of non-carboxyl group containing monomer units to carboxyl group containing monomer units in the polysiloxane is in the range from 40 to 150:1.

The invention also provides a sunscreen composition comprising particles of metal oxide having an average particle size of less from 200 nm a siloxane fluid and 1 to 60% by weight second on the weight of metal oxide of a polysiloxane comprising (i) in the range from 0.1 to 3 carboxyl groups, and (ii) non-carboxyl group containing monomer units to carboxyl group containing monomer units at a ratio in the range from 40 to 150:1.

The invention yet further provides the use of a dispersion comprising particles of zinc oxide dispersed in a siloxane fluid and 1 to 60% by weight based on the weight of metal oxide of a dispersing agent which is a polysiloxane wherein (i) the polysiloxane comprises in the range from 0.1 to 3 carboxyl groups, and (ii) the ratio of non-carboxyl group containing monomer units to carboxyl group containing monomer units in the polysiloxane is in the range from 40 to 150:1, to form an end-use sunscreen composition.

By dispersion is meant a true dispersion, i.e. where the solid particles are stable to aggregation. The particles in the dispersion are relatively uniformly dispersed and resistant to settling out on standing, but if some settling out does occur, the particles can be easily redispersed by simple agitation.

The polysiloxane dispersing agent is preferably a polyalkylsiloxane, wherein the alkyl groups are preferably lower alkyl, such as C₁₋₆, more preferably C₁₋₃, and particularly methyl groups.

By carboxyl group of the polysiloxane is meant a free functional carboxyl group and/or ester and/or salt derivative thereof. Suitable ester groups include lower alkyl, such as C₁₋₆, preferably C₁₋₃, and particularly methyl groups. Suitable salt derivatives include metal, such as alkali metal, preferably sodium or potassium. In a preferred embodiment of the invention, the carboxyl group of the-polysiloxane is in the form of the free acid and/or salt thereof, and more preferably in the form of the free acid.

The polysiloxane suitably comprises in the range from 0.5 to 3, preferably 0.8 to 2.5, more preferably 0.9 to 2, particularly 1 to 1.5, and especially 1.1 to 1.4 carboxyl groups (or carboxyl group containing monomer units) per molecule. In a preferred embodiment, a mixture of polysiloxane molecules containing different numbers of carboxyl groups are employed, and therefore the number of carboxyl groups per molecule is an average value (by number) and may be a non-integer.

In addition, the polysiloxane suitably comprises in the range from 20 to 500, preferably 30 to 200, more preferably 40 to 150, particularly 50 to 120, and especially 60 to 100 non-carboxyl group containing monomer units per molecule.

The ratio of non-carboxyl group containing monomer units to carboxyl group containing monomer units in the polysiloxane is preferably in the range from preferably 40 to 100:1, more preferably 45 to 75:1, particularly 50 to 70:1, and especially 55 to 65:1.

The carboxyl group(s) is preferably attached terminally and/or laterally, more preferably laterally, and particularly only laterally, to the polysiloxane chain. In an alternative embodiment, the carboxyl group is attached only terminally to the polysiloxane chain. The carboxyl group is suitably attached through a hydrocarbon linkage which may contain a hetero atom. In a preferred embodiment of the invention, the carboxyl group is attached through a hydrocarbon linkage which comprises a pyrrolidone group.

The polysiloxane suitably has a molecular weight (number average), preferably measured by gel permeation chromatography, in the range from 500 to 50,000, preferably 2,000 to 20,000, more preferably 4,000 to 15,000, particularly 5,000 to 10,000, and especially 6,000 to 8,000.

The polysiloxane suitably has a viscosity in the range from 0.05 to 150, preferably 0.1 to 50, more preferably 0.2 to 10, particularly 0.5 to 5, and especially 0.7 to 1.2 Pa.s.

The polysiloxane preferably has the formula (1) below: 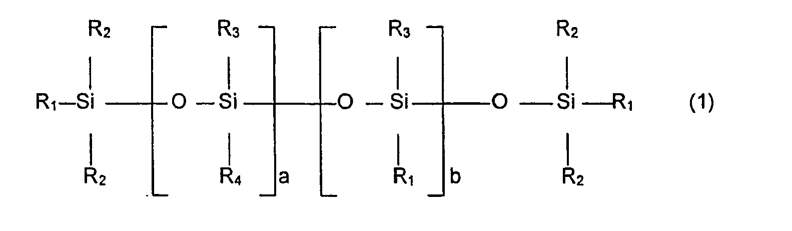 wherein: R₁, which can be the same or different, is selected from R₂, H, a primary amine containing group, and a pyrrolidone containing a carboxyl group and/or ester and/or salt derivative thereof of the formula (2): 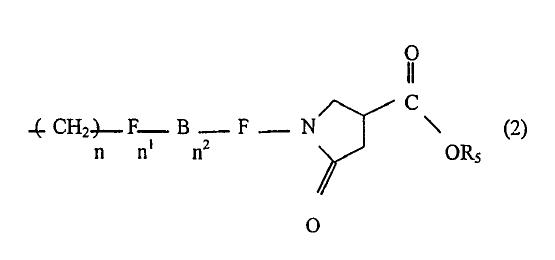 wherein 0.1 to 3 of the R₁ groups are of the formula (2);
F, can be the same or different, is linear or branched alkylene of 1-12 carbon atoms;
n is zero or 2; n¹ is zero or 1; n² is zero or 1; with the proviso that when n is 0 and
n² is 1, n¹ is 1, when n is 2 and n² is 1, n¹ is 0 or 1 and when n is 2 and n² is 0, n¹ is 0;
B is -NR₉, sulphur (S) or oxygen (O), wherein R₉ is hydrogen or lower alkyl (C₁₋₆), R₂ can be the same or different and is selected from alkyl, aryl and olefinic (vinyl);
R₃ and R₄, can be the same or different, are selected from alkyl, aryl, capped or uncapped polyoxyalkylene, alkaryl, aralkylene and alkenyl;
R₅ is hydrogen, lower alkyl (C₁₋₆) or a metal;
a which may be an integer, is in the range from 10 to 1,000; and
b which may be an integer, is in the range from 0.1 to 3, when the lateral R₁ group is of the formula (2).

In a preferred embodiment of the invention, R₅ is hydrogen or alkali metal, more preferably hydrogen. R₁ is preferably R₂ or a pyrrolidone containing a carboxyl group and/or ester and/or salt derivative thereof. At least one laterally linked R₁ group is preferably a pyrrolidone containing a carboxyl group and/or ester and/or salt derivative thereof. R₃ and R₄ are preferably alkyl, more preferably lower alkyl C₁₋₆, particularly C₁₋₃, and especially methyl.

Suitably a is in the range from 20 to 500, preferably 30 to 200, more preferably 40 to 150, particularly 50 to 120, and especially 60 to 100. In addition, when the lateral R₁ group of b is of the formula (2) above, then b is suitably in the range from 0.5 to 3, preferably 0.8 to 2.5, more preferably 0.9 to 2, particularly 1 to 1.5, and especially 1.1 to 1.4. The ratio of a:b is preferably in the range from 40 to 100:1, more preferably 45 to 75:1, particularly 50 to 70:1, and especially 55 to 65:1.

Both terminal R₁ groups are preferably R₂. R₂ is preferably alkyl, more preferably lower alkyl C₁₋₆, particularly C₁₋₃, and especially methyl. B is preferably - NR₉. In a particularly preferred embodiment of the invention, n is 2, n¹ is 0, n² is 0 and F is CH₂ or n is 2, n¹ is 0, n² is 1, B is NCH₃ and F is CH₂, and especially n is 2, n¹ is 0, n² is 0 and F is CH₂.

In a particularly preferred embodiment of the invention, the polysiloxane dispersing agent has the formula (3) below: 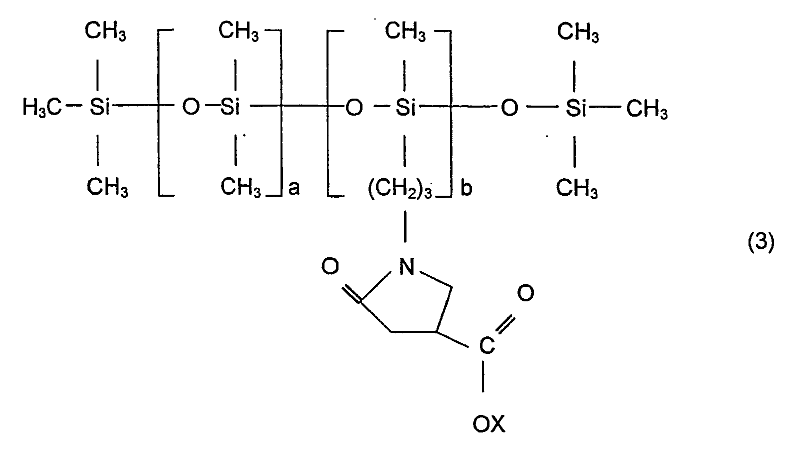 wherein X is hydrogen or alkali metal, a, b, and the ratio of a:b are as defined above.

US-5596061-A, the teaching of which is incorporated herein by reference, discloses suitable polysiloxanes containing a carboxyl group, and methods of synthesis thereof, for example by reacting a polysiloxane having a functional primary amine group with itaconic acid.

The amount of polysiloxane dispersing agent present in the dispersion according to the present invention is preferably in the range from 1 to 60%, more preferably 3 to 40%, particularly 5 to 30%, and especially 6 to 25% by weight based on the weight of particles of metal oxide.

Preferably the metal oxide used in the invention comprises an oxide of titanium, zinc or iron, more preferably is titanium dioxide or zinc oxide, and particularly zinc oxide.

The average primary particle size of the particles of metal oxide is preferably less than 200 nm, and where the particles are substantially spherical then this size will be taken to represent the diameter. However, the invention also encompasses particles of metal oxides which are non-spherical and in such cases the average primary particle size refers to the largest dimension. The average particle size which characterises the metal oxides used in the invention is the average size of primary particles, this average size typically being determined by electron microscopy. The size therefore relates to particles of metal oxide which are not aggregated. Frequently, the primary particles consist of single crystals but may also comprise several crystals fused together.

Preferably the average primary particle size of the particles is in the range from 5 to 150 nm, and more preferably 10 to 100 nm when they are substantially spherical. For titanium dioxide particles having an acicular shape the average largest dimension of the primary particles is preferably less than 150 nm, and more preferably in the range from 20 to 100 nm.

When the metal oxide is titanium dioxide the particles are preferably acicular in shape and have a ratio of largest dimension to shortest dimension in the range from 10:1 1 to 2:1. Also, when the metal oxide is titanium dioxide the amount of polysiloxane dispersing agent present in the dispersion is preferably in the range 5 to 60%, more preferably 9 to 40%, particularly 12 to 30%, and especially 15 to 25% by weight based on the weight of particles of titanium dioxide.

When the metal oxide is zinc oxide the particles suitably have an average primary particle size in the range from 30 to 100 nm, and preferably 60 to 90 nm. Also, when the metal oxide is zinc oxide the amount of polysiloxane dispersing agent present in the dispersion is suitably in the range 1 to 25%, preferably 3 to 18%, more preferably 5 to 12%, particularly 6 to 10%, and especially 7 to 9% by weight based on the weight of particles of zinc oxide.

The particles of metal oxide may comprise substantially pure metal oxide but preferably also carry an inorganic coating. For example, particles of titanium dioxide can be coated with oxides of other elements such as oxides of aluminium, zirconium or silicon and a form of acicular titanium dioxide, coated with alumina and silica, which is especially useful in the process of this invention is disclosed in GB-2205088-A. Alternatively, particulate metal oxides which carry alumina as the only inorganic oxide coating have also been found to be useful in this invention. The preferred amount of inorganic coating is in the range 4 to 20% by weight, calculated as inorganic oxide with respect to weight of metal oxide core particles. More preferably, the amount of inorganic coating is in the range from 5 to 15% by weight, calculated as inorganic oxide with respect to weight of metal oxide core particles. Suitable inorganic coatings can be applied using any appropriate technique and a person skilled in the art will readily be able to apply such a technique. A typical process comprises forming an aqueous dispersion of metal oxide core particles in the presence of a soluble salt of the inorganic element whose oxide will form the coating. This dispersion is usually acidic or basic, depending upon the nature of the salt chosen, and precipitation of the inorganic oxide is achieved by adjusting the pH of the dispersion by the addition of acid or alkali, as appropriate.

In a preferred embodiment, the metal oxide particles used to form dispersions of the invention are hydrophobic. The metal oxide particles may be rendered hydrophobic, for example, by application of a hydrophobic coating on the surface of the metal oxide particles. The hydrophobic coating may be applied prior to formation of the dispersion, or alternatively in situ, ie during dispersion formation. In addition, as described above, the particles may carry an inorganic coating. Therefore, the term "particles of metal oxide", as used herein is taken to mean the complete particles, i.e. the core particles plus any coating which has been applied.

Generally, the metal oxide particles are treated with a water-repellent material in order to render them hydrophobic. Suitable water-repellent materials include fatty acids, preferably fatty acids containing 10 to 20 carbon atoms, such as lauric acid, stearic acid and isostearic acid, salts of the above fatty acids such as sodium salts and aluminium salts, fatty alcohols, such as stearyl alcohol, and silicones such as polydimethylsiloxane and substituted polydimethylsiloxanes and reactive silicones such as methylhydrosiloxane polymers and copolymers.

The hydrophobic treatment can be applied using any conventional process. Typically, metal oxide core particles (uncoated or with an inorganic coating) are dispersed in water and heated to a temperature in the range 50 to 80°C. A fatty acid is then deposited on the metal oxide particles by adding a salt of the fatty acid (e.g. sodium stearate) to the dispersion, followed by an acid. Alternatively, the metal oxide core particles or inorganically coated core particles can be mixed with a solution of the water-repellent material in an organic solvent, followed by evaporation of the solvent. In an alternative and preferred embodiment of the invention, the water-repellant material can be added directly to the dispersion of the present invention, during preparation thereof, such that the hydrophobic coating is formed in situ.

Generally, the particles are treated with up to 20% by weight of the water-repellent material, calculated with respect to the coated or uncoated core particles, as appropriate, and preferably in the range from 0.05 to 3% by weight of water-repellent material, calculated with respect to coated or uncoated core particles.

The dispersion according to the present invention suitably contains greater than 30%, preferably greater than 40%, more preferably greater than 45%, particularly greater than 50%, and especially greater than 60% by weight of particles of metal oxide, particularly zinc oxide, based on the total weight of the dispersion. Generally it is difficult to produce dispersions containing greater than 75% by weight of particles of metal oxide.

The metal oxide used in the dispersion of the invention is dispersed in a siloxane fluid dispersing medium. Any suitable siloxane fluid can be used, a principal requirement being cosmetic acceptability. One preferred type of siloxane fluid is a cyclic oligomeric dialkylsiloxane, such as the cyclic pentamer of dimethylsiloxane known as cyclomethicone. Alternative fluids include dimethylsiloxane linear oligomers or polymers having a suitable fluidity and phenyltris(trimethylsiloxy)silane (also known as phenyltrimethicone).

The dispersion may further contain conventional additives suitable for use in the intended application for the dispersion, such as conventional cosmetic ingredients used in sunscreens. Preferably, for maximum flexibility of use, the dispersion consists essentially of the specified ingredients (particles of metal oxide, siloxane fluid dispersing medium and carboxyl group containing polysiloxane dispersing agent).

The dispersions of the invention are particularly useful for preparing sunscreen products and other compositions intended to attenuate ultraviolet light. For such applications it is desirable that the dispersion strongly attenuates ultraviolet light but in use, i.e. when applied to the skin in a sunscreen formulation, is substantially transparent to visible light. When the metal oxide is titanium dioxide the dispersions preferably have a maximum extinction coefficient for light in the ultraviolet range of wavelengths of at least 40, more preferably at least 50 litres per gram per cm. When the metal oxide is zinc oxide the dispersions preferably have a maximum extinction coefficient for light in the ultraviolet range of wavelengths of at least 15, more preferably at least 20 litres per gram per cm.

The particles of metal oxide in a dispersion which in use is substantially transparent to visible light will have an extinction coefficient for light in the visible range of wavelengths preferably not greater than 10, more preferably not greater than 5, and particularly not greater than 2 litres per gram per cm.

The dispersions of metal oxides according to the present invention are preferably prepared by milling with a particulate grinding medium. A suitable mill which is employed to effect the grinding of the metal oxide product in the dispersing medium is one which uses a particulate grinding medium to grind the product. Typical of such mills are bead mills equipped with one or more agitators and using sand, glass beads or ceramic beads or other particles as the particulate grinding medium. Particularly useful are those mills which operate at a high speed and, depending on the size of mill, a speed in the range 500 to 5000 rev. per minute (r.p.m) is generally suitable. Preferably, mills operating at a speed in the range 800 r.p.m to 3000 r.p.m are used. Agitator mills in which the tip speed of the agitator is up to and can exceed 10 metres per second are of use. If desired the mill can be cooled. Generally, the dispersions are pre-mixed before milling using a high speed stirrer, but, in an alternative process, the dispersing medium is added to the mill initially and then the metal oxide and the dispersing agent co-added to the dispersing medium subsequently. After milling has been carried out for the required time the dispersion is separated from the grinding medium by screening through a narrow gap.

Generally, this method can be used to prepare dispersions possessing the properties of the dispersions of the invention mentioned hereinbefore. In particular, dispersions which strongly attenuate ultraviolet light but in use are substantially transparent to visible light can be prepared using the method. Frequently, it is possible to adjust the light attenuating profile by adjusting the conditions (e.g. length of time, proportion of grinding medium, concentration of dispersing agent or metal oxide) under which the milling is carried out.

The dispersions of the invention are useful as ingredients for preparing end-use sunscreen compositions. The amount of metal oxide present in the sunscreen composition is preferably in the range from 0.5 to 40%, and more preferably 1.0 to 30%, and particularly 2.0 to 20% by weight of the composition. The metal oxide particles may provide the only ultraviolet light attenuators in the sunscreen composition, but other sunscreening agents, particularly organic sunscreens, may also be added.

The invention is illustrated by the following examples.

The following test procedures were employed;
i) ²⁹Si NMR was used to measure the number of non-carboxyl group containing monomer units (e.g. a in formulae (1) and (3)), the number of carboxyl group containing monomer units (e.g. formula (2) and b in formula (3)), the a:b ratio, and the molecular weight of the polysiloxane dispersing agent.
ii) Viscosity was measured with a Brookfield RVT viscometer using an appropriate spindle at 10 rpm and 25°C, and results are quoted in Pa.s.

### Example 1

86.3 g of cyclomethicone, 11.3 g of polysiloxane containing a carboxyl group (Monasil PCA (trade mark, ex Uniqema)) and 150 g of hydrophobically coated zinc oxide having an average primary particle size of approximately 80 nm (calculated from specific surface area) were mixed together and thoroughly agitated for 15 minutes. The mixture was then passed through a horizontal bead mill operating at approximately 1500 r.p.m. and containing zirconia beads as grinding media. The mixture was passed through the mill and was then returned to the pre-mix vessel and passed through the mill a further three times. The optical characteristics of the resulting dispersion (as determined by UV spectrometry after dilution by a factor of 20,000 using n-octanol) are given in Table 1 below, where λₘₐₓ is the wavelength in nm at which maximum attenuation was observed and Eₘₐₓ, E₃₀₈, E₃₆₀ and E₅₂₄ are the observed extinction coefficients in I/g/cm at λₘₐₓ, 308 nm, 360 nm and 524 nm respectively.

**Table 1**

| λₘₐₓ | Eₘₐₓ | E₃₀₈ | E₃₆₀ | E₅₂₄ |
|---|---|---|---|---|
| 374 | 17.0 | 13.3 | 14.2 | 2.8 |

### Example 2

The procedure of Example 1 was repeated except that 66.8 g of cyclomethicone, 10.8 g of Monasil PCA, 2.4 g isostearic acid (Prisorine 3505 (trade mark, ex Uniqema)) and 120 g of uncoated zinc oxide having an average primary particle size of approximately 60 nm were used. The optical characteristics of the resulting dispersion were determined by diluting by a factor of 20,000 in cyclohexane. The results are given in Table 2 below

**Table 2**

| λₘₐₓ | Eₘₐₓ | E₃₀₈ | E₃₆₀ | E₅₂₄ |
|---|---|---|---|---|
| 367 | 15.6 | 14.1 | 14.9 | 1.3 |

This dispersion was used to prepare a sunscreen formulation having the following composition;

| Phase A | % by wt. |
|---|---|
| ARLACEL P135 (trade mark, ex Uniqema) | 2.0 |
| ARLAMOL HD P135 (trade mark, ex Uniqema) | 5.0 |
| ARLAMOL E P135 (trade mark, ex Uniqema) | 2.4 |
| Finsolv TN | 4.0 |
| Candelilla Wax | 1.0 |
| Magnesium Stearate | 0.7 |
| Zinc oxide dispersion produced in Example 1 | 14.0 |
| Jojoba Oil | 4.0 |
| Cyclomethicone | 5.6 |
| Parsol MCX | 8.0 |

| Phase B | |
|---|---|
| ATLAS G-2330 (trade mark, ex Uniqema) | 3.0 |
| Germaben II | 1.0 |
| Magnesium Sulphate | 0.7 |
| D-Panthanol USP | 0.8 |
| Allantoin | 0.2 |
| Demineralised Water | 47.6 |

### Procedure

(i) Phases A and B were prepared separately and heated to 75-80°C.
(ii) Phase B was slowly added to phase A with intensive stirring using a Silverson mixer for two minutes.
(iii) The mixture was cooled to 25'C with intensive stirring.
The Sun Protection Factor for the product was determined using the *in vitro* method of Diffey and Robson, J. Soc. Cosmet. Chem. Vol. 40, pp 127-133,1989, and a value of 23 was obtained.

The above examples illustrate the improved properties of a dispersion and sunscreen according to the present invention.

## Claims

1. A dispersion comprising particles of metal oxide having an average primary particle size of less than 200 nm, dispersed in a siloxane fluid and 1 to 60% by weight, based on the weight of the metal oxide, of a dispersing agent which is a polysiloxane wherein (i) the polysiloxane comprises in the range from 0.1 to 3 carboxyl groups, and (ii) the ratio of non-carboxyl group containing monomer units to carboxyl group containing monomer units in the polysiloxane is in the range from 40 to 150:1.

2. A dispersion according to claim 1 wherein the polysiloxane has a viscosity in the range from 0.2 to 10 Pa.s.

3. A dispersion according to either one of claims 1 and 2 wherein the polysiloxane has a molecular weight (number average) in the range from 4,000 to 15,000.

4. A dispersion according to any one of the preceding claims wherein the dispersion comprises greater than 30%, more preferably greater than 40%, and particularly greater than 50% by weight of particles of metal oxide.

5. A dispersion according to any one of the preceding claims wherein the metal oxide is titanium dioxide.

6. A dispersion according to any one of the preceding claims wherein the metal oxide is zinc oxide.

7. A dispersion according to any one of the preceding claims wherein the polysiloxane comprises 0.8 to 2.5 carboxyl groups per molecule.

8. A dispersion according to any one of the preceding claims wherein the polysiloxane comprises in the range from 30 to 200 non-carboxyl group containing monomer units.

9. A dispersion according to any one of the preceding claims wherein the carboxyl group is attached laterally, preferably only laterally, to the polysiloxane chain.

10. A dispersion according to any one of the preceding claims wherein the metal oxide particles are hydrophobic.

11. A dispersion according to any one of the preceding claims wherein the siloxane fluid dispersing medium is a cyclic oligomeric dialkylsiloxane, a linear dimethylsiloxane oligomer and/or polymer, and/or phenyltris(trimethylsiloxy)silane.

12. A dispersion according to any one of the preceding claims consisting of metal oxide, siloxane fluid and dispersing agent.

13. A dispersion according to any one of the preceding claims wherein the average primary particle size of the metal oxide particles is 5 to 150 nm.

14. A dispersion according to any one of the preceding claims wherein the particles of metal oxide have an extinction coefficient for light in the visible wavelengths of not greater than 10 litres per gram per cm.

15. A method of preparing a dispersion of metal oxide which comprises milling with a particulate grinding medium particles of metal oxide in a siloxane fluid in the presence of 1 to 60% by weight, based on the weight of the metal oxide, of a dispersing agent which is a polysiloxane wherein (i) the polysiloxane comprises in the range from 0.1 to 3 carboxyl groups, and (ii) the ratio of non-carboxyl group containing monomer units to carboxyl group containing monomer units in the polysiloxane is in the range from 40 to 150:1.

16. A sunscreen composition comprising particles of metal oxide having an average primary particle size of less than 200 nm, a siloxane fluid, and 1 to 60% by weight, based on the weight of the metal oxide, of a polysiloxane comprising (i) in the range from 0.1 to 3 carboxyl groups, and (ii) non-carboxyl group containing monomer units to carboxyl group containing monomer units at a ratio in the range from 40 to 150:1.

17. The use of a dispersion comprising particles of zinc oxide dispersed in a siloxane fluid and 1 to 60% by weight, based on the weight of the metal oxide, of a dispersing agent which is a polysiloxane wherein (i) the polysiloxane comprises in the range from 0.1 to 3 carboxyl groups, and (ii) the ratio of non-carboxyl group containing monomer units to carboxyl group containing monomer units in the polysiloxane is in the range from 40 to 150:1, to form an end-use sunscreen composition.

## Patentansprüche

1. Dispersion, enthaltend Teilchen aus Metalloxid mit einer durchschnittlichen Primärteilchengröße von weniger als 200 nm, dispergiert in einem Siloxanfluid und 1 bis 60 Gew.-%, basierend auf dem Gewicht des Metalloxids, eines Dispersionsagens, welches ein Polysiloxan ist, wobei
(i) das Polysiloxan in dem Bereich von 0,1 bis 3 Carboxylgruppen enthält, und
(ii) das Verhältnis von Nicht-Carboxylgruppeenthaltenden Monomereinheiten zu Carboxylgruppeenthaltenden Monomereinheiten in dem Polysiloxan in dem Bereich von 40 bis 150:1 ist.

2. Dispersion nach Anspruch 1, wobei das Polysiloxan eine Viskosität in dem Bereich von 0,2 bis 10 Pa.s hat.

3. Dispersion nach einem der Ansprüche 1 und 2, wobei das Polysiloxan ein Molekulargewicht (Zahlenmittel) in dem Bereich von 4.000 bis 15.000 hat.

4. Dispersion nach einem der vorhergehenden Ansprüche, wobei die Dispersion mehr als 30 Gew.-%, vorzugsweise mehr als 40 Gew.-%, und insbesondere mehr als 50 Gew.-% an Teichen aus Metalloxid aufweist.

5. Dispersion nach einem der vorhergehenden Ansprüche, wobei das Metalloxid Titandioxid ist.

6. Dispersion nach einem der vorhergehenden Ansprüche, wobei das Metalloxid Zinkoxid ist.

7. Dispersion nach einem der vorhergehenden Ansprüche, wobei das Polysiloxan 0,8 bis 2,5 Carboxylgruppen pro Molekül enthält.

8. Dispersion nach einem der vorhergehenden Ansprüche, wobei das Polysiloxan in dem Bereich von 30 bis 200 Nicht-Carboxylgruppe-enthaltende Monomereinheiten enthält.

9. Dispersion nach einem der vorhergehenden Ansprüche, wobei die Carboxylgruppe seitlich, vorzugsweise nur seitlich, an die Polysiloxankette angebracht ist.

10. Dispersion nach einem der vorhergehenden Ansprüche, wobei die Metalloxidteilchen hydrophob sind.

11. Dispersion nach einem der vorhergehenden Ansprüche, wobei das Siloxanfluid-Dispersionsmedium ein zyklisches oligomeres Dialkylsiloxan, ein lineares Dimethylsiloxan-Oligomer und/oder -Polymer, und/oder Phenyltris(Trimethylsiloxy)silan ist.

12. Dispersion nach einem der vorhergehenden Ansprüche, bestehend aus Metalloxid, Siloxanfluid und Dispersionsagens.

13. Dispersion nach einem der vorhergehenden Ansprüche, wobei die durchschnittliche Primärteilchengröße der Metalloxidteilchen 5 bis 150 nm ist.

14. Dispersion nach einem der vorhergehenden Ansprüche, wobei die Teilchen aus Metalloxid einen Extinktionskoeffizienten für Licht in den sichtbaren Wellenlängen von nicht mehr als 10 Liter pro Gramm pro cm aufweisen.

15. Verfahren des Bereitstellens einer Metalloxid-Dispersion, welches beinhaltet das Zerkleinern mit einem partikulären Mahlmedium von Teilchen aus Metalloxid in einem Siloxanfluid in der Gegenwart von 1-60 Gew.%, basierend auf dem Gewicht des Metalloxids, eines Dispersionsagens, welches ein Polysiloxan ist, wobei
(i) das Polysiloxan in dem Bereich von 0,1 bis 3 Carboxylgruppen enthält, und
(ii) das Verhältnis von Nicht-Carboxylgruppeenthaltenden Monomereinheiten zu Carboxylgruppeenthaltenden Monomereinheiten in dem Polysiloxan in dem Bereich von 40 bis 150:1 ist.

16. Sonnenschutz-Zusammensetzung, enthaltend Teilchen aus Metalloxid mit einer durchschnittlichen Primärteilchengröße von weniger als 200 nm, ein Siloxanfluid, und 1 bis 60 Gew.-%, basierend auf dem Gewicht des Metalloxids, eines Polysiloxans, welches enthält
(i) in dem Bereich von 0,1 bis 3 Carboxylgruppen, und
(ii) Nicht-Carboxylgruppe-enthaltende Monomereinheiten zu Carboxylgruppe-enthaltende Monomereinheiten in einem Verhältnis in dem Bereich von 40 bis 150:1 ist.

17. Verwendung einer Dispersion, enthaltend Teilchen aus Zinkoxid, dispergiert in einem Siloxanfluid und 1 bis 60 Gew.-%, basierend auf dem Gewicht des Metalloxids, eines Dispersionsagens, welches ein Polysiloxan ist, wobei
(i) das Polysiloxan in dem Bereich von 0,1 bis 3 Carboxylgruppen aufweist, und
(ii) das Verhältnis von Nicht-Carboxylgruppeenthaltenden Monomereinheiten zu Carboxylgruppeenthaltenden Monomereinheiten in dem Polysiloxan in dem Bereich von 40 bis 150:1 ist, um eine Endverbrauchs-Sonnenschutz-Zusammensetzung zu bilden.

## Revendications

1. Dispersion comprenant des particules d'oxyde métallique ayant une taille de particules primaires moyenne inférieure à 200 nm, dispersées dans un fluide de siloxane et 1 à 60 % en poids sur la base du poids de l'oxyde métallique, d'un agent dispersant qui est un polysiloxane, dans laquelle (i) le polysiloxane comprend de l'ordre de 0,1 à 3 groupes carboxyle et (ii) le rapport des motifs monomères contenant des groupes non carboxyle aux motifs de monomère contenant des groupes carboxyle dans le polysiloxane est de 40 à 150:1.

2. Dispersion selon la revendication 1, dans laquelle le polysiloxane a une viscosité de l'ordre de 0,2 à 10 Pa.s.

3. Dispersion selon l'une ou l'autre des revendications 1 et 2, dans laquelle le polysiloxane a un poids moléculaire (moyenne en nombre) de l'ordre de 4000 à 15 000.

4. Dispersion selon l'une quelconque des revendications précédentes, la dispersion comprenant plus de 30 %, de manière davantage préférée, plus de 40 % et en particulier, plus de 50 % en poids de particules d'oxyde métallique.

5. Dispersion selon l'une quelconque des revendications précédentes, dans laquelle l'oxyde métallique est le dioxyde de titane.

6. Dispersion selon l'une quelconque des revendications précédentes, dans laquelle l'oxyde métallique est l'oxyde de zinc.

7. Dispersion selon l'une quelconque des revendications précédentes, dans laquelle le polysiloxane comprend 0,8 à 2,5 groupes carboxyle par molécule.

8. Dispersion selon l'une quelconque des revendications précédentes, dans laquelle le polysiloxane comprend de l'ordre de 30 à 200 motifs de monomère contenant des groupes non carboxyle.

9. Dispersion selon l'une quelconque des revendications précédentes, dans laquelle le groupe carboxyle est lié latéralement, de préférence uniquement latéralement à la chaîne polysiloxane.

10. Dispersion selon l'une quelconque des revendications précédentes, dans laquelle les particules d'oxyde métallique sont hydrophobes.

11. Dispersion selon l'une quelconque des revendications précédentes, dans laquelle le milieu de dispersion du fluide de siloxane est un dialkylsiloxane oligomère cyclique, un oligomère de diméthylsiloxane linéaire et/ou un polymère et/ou le phényltris(triméthylsiloxy)silane.

12. Dispersion selon l'une quelconque des revendications précédentes, constituée d'oxyde métallique, de fluide de siloxane et d'agent dispersant.

13. Dispersion selon l'une quelconque des revendications précédentes, dans laquelle la taille des particules primaires moyenne des particules d'oxyde métallique est de 5 à 150 nm.

14. Dispersion selon l'une quelconque des revendications précédentes, dans laquelle les particules d'oxyde métallique ont un coefficient d'extinction de la lumière dans les longueurs d'ondes visibles qui ne dépasse pas 10 litres par gramme par cm.

15. Procédé de préparation d'une dispersion d'oxyde métallique qui comprend le broyage avec un milieu de broyage de particules, de particules d'oxyde métallique dans un fluide de siloxane en présence de 1 à 60 % en poids sur la base du poids de l'oxyde métallique d'un agent dispersant qui est un polysiloxane, dans laquelle (i) le polysiloxane comprend de l'ordre de 0,1 à 3 groupes carboxyle et (ii) le rapport des motifs monomères contenant des groupes non carboxyle aux motifs de monomère contenant des groupes carboxyle dans le polysiloxane est de 40 à 150:1.

16. Composition d'écran solaire comprenant des particules d'oxyde métallique ayant une taille de particules primaires moyenne inférieure à 200 nm, un fluide de siloxane et 1 à 60 % en poids sur la base du poids de l'oxyde métallique d'un polysiloxane comprenant (i) de l'ordre de 0,1 à 3 groupes carboxyle et (ii) le rapport des motifs monomères contenant des groupes non carboxyle aux motifs de monomère contenant des groupes carboxyle dans le polysiloxane est de 40 à 150:1.

17. Utilisation d'une dispersion comprenant des particules d'oxyde de zinc dispersées dans un fluide de siloxane et 1 à 60 % en poids sur la base du poids de l'oxyde métallique d'un agent dispersant qui est un polysiloxane, (i) le polysiloxane comprenant de l'ordre de 0,1 à 3 groupes carboxyle et (ii) le rapport des motifs monomères contenant des groupes non carboxyle aux motifs de monomère contenant des groupes carboxyle dans le polysiloxane étant de 40 à 150:1, pour former une composition d'écran solaire en utilisation finale.
